# EUROPÄISCHE PATENTSCHRIFT

(11) **EP 1 315 972 B1**
(45) Veröffentlichungstag und Bekanntmachung des Hinweises auf die Patenterteilung: **11.01.2006**
(21) Anmeldenummer: 01967313.6
(22) Anmeldetag: 06.09.2001
(51) Int. Cl.: G01N 33/68

(54) **VERFAHREN ZUR DETEKTION DER AKTIVITÄT VON WIRKSTOFFEN**
METHOD FOR DETECTING THE ACTIVITY OF ACTIVE INGREDIENTS
PROCEDE DE DETECTION DE L'ACTIVITE DE SUBSTANCES ACTIVES

(30) Priorität: 07.09.2000 DE 10045808
(43) Veröffentlichungstag der Anmeldung: 04.06.2003
(73) Patentinhaber: IMB Institut für Molekulare Biotechnologie e.V., 07745 Jena (DE)
(72) Erfinder: BÖHM, Konrad, 07747 Jena (DE); NEUMANN, Tobias, 56242 Selters (DE); UNGER, Eberhard, 07751 Jena-Cospeda (DE)
(74) Vertreter: Bock, Gerhard
(86) Internationale Anmeldenummer: PCT/EP2001/010291
(87) Internationale Veröffentlichungsnummer: WO 2002/021126

(56) Entgegenhaltungen:
- WO-A-98/55866
- VON MASSOW A ET AL: "INTERACTION BETWEEN KINESIN MICROTUBULES AND MICROTUBULE-ASSOCIATED PROTEIN 2" CELL MOTILITY AND THE CYTOSKELETON, Bd. 14, Nr. 4, 1989, Seiten 562-571, XP008007379 ISSN: 0886-1544
- SAKOWICZ R ET AL: "A MARINE NATURAL PRODUCT INHIBITOR OF KINESIN MOTORS" SCIENCE, AMERICAN ASSOCIATION FOR THE ADVANCEMENT OF SCIENCE,, US, Bd. 280, 10. April 1998 (1998-04-10), Seiten 292-295, XP002211157 ISSN: 0036-8075 in der Anmeldung erwähnt
- STEWART RUSSELL J ET AL: "Direction of microtuble movement is an intrinsic property of the motor domains of kinesin heavy chain and Drosophila ncd protein." PROCEEDINGS OF THE NATIONAL ACADEMY OF SCIENCES OF THE UNITED STATES, Bd. 90, Nr. 11, 1993, Seiten 5209-5213, XP002211158 1993 ISSN: 0027-8424
- WOOD KENNETH W ET AL: "Past and future of the mitotic spindle as an oncology target." CURRENT OPINION IN PHARMACOLOGY, Bd. 1, Nr. 4, August 2001 (2001-08), Seiten 370-377, XP002211159 August, 2001 ISSN: 1471-4892

## Beschreibung

Die Erfindung betrifft ein Verfahren zur Identifikation von Kinesin-spezifischen Wirkstoffen, das insbesondere bei der Suche nach Inhibitoren der Kinesin-Last- und Kinesin-Mikrotubulus-Bindungen Verwendung findet.

Es ist bekannt, daß filamentöse oder röhrenförmige Proteinassemblate, die über Bindungsstellen für Motorproteine verfügen, wie z.B. Mikrotubuli, als Bestandteile des Zytoskeletts lebender eukaryotischer Zellen gemeinsam mit mechanochemischen Proteinen, wie z.B. Kinesin, die auch als Motorproteine bezeichnet werden, verschiedene Funktionen bei zellulären Bewegungsvorgängen, wie z.B. beim Vesikeltransport, bei der Zellteilung und bei der Zellbewegung, erfüllen.

Die Mikrotubuli haben Durchmesser von etwa 25 nm und Längen von einigen Mikrometern. Sie stellen Leitbahnen dar, an denen sich Motorproteinmoleküle, wie z.B. Kinesin, entlang bewegen. Die Energie für die Bewegung wird aus der Hydrolyse von Nukleotidtriphosphaten, vorzugsweise Adenosintriphophat (ATP), durch Umwandlung von chemischer in mechanische Energie geliefert (Kuznetsov S.A., Gelfand V.I., 1986: Bovine brain kinesin is a microtubule-activated ATPase. Proc. Natl. Acad. Sci. USA, 83, 8530-8534; Cohn S.A., Ingold A.L., Scholey J.M., 1989: Quantitative analysis of sea urchin egg kinesin-driven microtubule motility. J. Biol. Chem., 264, 4290-4297).

Die Kinesine sind Proteine, die innerhalb ihrer Aminosäuresequenz verschiedene Domänen aufweisen, denen verschiedene Funktionen zuordenbar sind und die zum einen konservativ und zum anderen auch variabel sein können. Sowohl zwischen verschiedenen eukaryotischen Zelltypen als auch zwischen verschiedenen Spezies sind Sequenz- und

Funktionshomologien bekannt. Dabei sind die s.g. Motordomänen, die die Mikrotubulusbindungsdomänen und die ATP-Bindungsdomänen enthalten in ihrer Sequenz hoch konservativ. Für die s.g. Vesikelbindungsdomäne hingegen sind größere Sequenzvariationen bekannt (Conforti, L.; Buckmaster E.A.: Tarlton, A.; Brown, M.C.; Lyon, M.F. and M.P. Coleman; Mamm Genome 1999, 10: 617-622).

Die Vesikelbindungsstelle stellt einen Bereich innerhalb des Kinesinmoleküles dar, der die Eigenschaft besitzt, unterschiedliche Zellorganellen (z.B. Vesikel und Zentomer) zu binden, was in direkten Zusammenhang mit der Sequenzvariabilität zu sehen ist und was diesen Molekülbereich zu einem interessanten Target für Wirkstoffe macht.

Aufgrund der verschiedenen Bindungsstellenpartner wird die Vesikelbindungsstelle auch verallgemeinernd als Lastbindungsstelle bezeichnet, was im vorliegenden Text auch der Fall ist.

Es gehört zu Stand der Technik, die Mikrotubuli-Kinesin-Bewegungsvorgänge auch unter in vitro-Bedingungen ablaufen zu lassen. Entweder bewegen sich dabei kleine Partikel (z.B. Latexkugeln), die mit Motorproteinmolekülen, wie z.B. Kinesin, bedeckt sind, entlang von immobilisierten Mikrotubuli (Wang Z. H.; Khan S.; Sheetz M., 1995: Single cytoplasmic dynein molecule movements: Characterization and comparison with kinesin. Biophysical Journal, 69, 2011-2023) oder es bewegen sich, in Umkehrung der Verhältnisse, die Mikrotubuli zufallsverteilt über Motorproteinmoleküle, die auf einem Träger gebunden sind (Vale R.D., Reese T.S., Sheetz M.P., 1985: Identification of a novel force-generating protein, kinesin, involved in microtubule-based motility. Cell , 42, 39-50).

Bei letzterem, dem sogenannten Gleitassay, sind die Kinesinmoleküle als Motorprotein auf einem Träger, z.B. Glas, gebunden und die zugesetzten Mikrotubuli werden durch die Aktivität des Kinesins in ihrer Längsrichtung richtungsorientiert vorwärtsbewegt (Vale R.D., Reese T.S., Sheetz M.P., 1985: Identification of a novel force-generating protein, kinesin, involved in microtubule-based motility. Cell, 42,39-50).

Auch ist bekannt, daß die Mikrotubuli, die einen polaren Charakter besitzen, der sich darauf begründet, daß die Mikrotubuli aus parallel zueinander angeordneten Protofilamenten, die wiederum aus polaren Ketten von Tubulin-Dimeren bestehen, aufgebaut sind. In Folge dieser Polarität bewegen sich Motorproteine, z.B. Kinesin, typischerweise nur in einer Richtung auf dem Mikrotubulus entlang. Der selbständige Zusammenfindungsprozeß der einzelnen Dimere findet in vivo, aber auch in vitro, in flüssiger Umgebung statt, Bei dem in vitro Prozeß können die Mikrotubuli in der Phase ihres Aufbaus in elektrischen Feldern parallelisiert werden (Vassiliv, P. M., Dronzine, R. T., Vassiliva, M.P. and G. A. Georgiev, 1982: Parallel arrays of microtubules formed in electric and magnetic fields. Bioscience Reports, 2: 1025-1029) bzw. sie können anhand markerter Tubulin-Dimere markiert werden.

Weiterhin ist bekannt, daß die Markierung von Biomolekülen, wie z.B. auch von Mikrotubuli oder Kinesinmolekülen, durch den chemischen oder enzymatischen Einbau von Radioisotopen, wie bspw. Tritium, Schwefel-35 oder Phosphor-32, von nichtradioaktiven Molekülen, wie bspw. Digoxigenin oder Biotin bzw. von nichtradioaktiven, fluoreszierenden Molekülen, wie bspw. Fluoresceinisothiocyanat oder 7-Amino-4-methylcumarin-3-acetat oder von metallischen Partikeln, wie bspw. Gold, erfolgen kann (Nicholl, D., S., T., 1995: Genetische Methoden, Spektrum Akademischer Verlag Heidelberg, S.24-27).

Darüber hinaus ist bekannt, daß die Detektion der Anwesenheit bioaktiver Moleküle in Form von Antigenen, wie bspw. Peptiden oder Proteinen, mit spezifischen und markierten Antikörpern erfolgen kann. Die Markierung der Antikörper erfolgt dabei durch Ankoppeln von Radioisotopen (z.B. Iod-125 oder Tritium) an Tyrosin- bzw. Histidinreste oder durch Ankoppeln von nichtradioaktiven Enzymen, wie bspw. alkalische Phosphatase oder Peroxidase, wobei die enzymatische Aktivität, bspw. durch die Umsetzung eines farblosen in ein farbiges Produkt, gemessen wird, oder durch Ankoppeln von nichtradioaktiven Enzymen, wie bspw. Hämatin, das die chemilumineszente Reaktion von Wasserstoffperoxid und Luminol bewirkt, oder durch Ankoppeln von nichtradioaktiven Enzymen, wie bspw. Luciferase, die Bioluminiszenz mittels phosphorilierten Luciferin bewirkt, oder durch das Ankoppeln von metallische Partikeln, wie bspw. Gold (Liddell, E. und Weeks, I: 1996: Antikörpertechniken, Spektrum Akademischer Verlag Heidelberg, S.87-107).

Die Signale der verwendeten verschiedenen Markermoleküle werden durch radio- oder elektrochemische, optische, piezoelektrische oder kalorimetrische Verfahren zur Darstellung von molekularen Erkennungsereignissen ausgewertet. Die Größe der Einzelsignalaussendenden Markermoleküle liegt dabei im Nanometerbereich.

Die meisten der derzeit verfügbaren und verwendeten Biomolekül-Nachweistechnologien beruhen auf der Detektion von fluoreszenzmarkierten Bindungspaaren, die in einer spezifischen Art auf der Oberfläche festgehalten sind, wobei die Fluoreszenzdetektion durch optisches Auslesen der reaktiven Zentren des Arrays ausgeführt wird. Der Gebrauch von fluoreszierenden oder chemilumeniszierenden Proben findet dabei wie in den zuvor beschriebenen klassischen Methoden Anwendung und wird mit CCD-Imaging kombiniert (Eggers, M. et al., 1996: Professional Program Proceedings. Electro '96. IEEE, New York, NY, USA, 364pp.; Heller, M.J., 1996: IEEE-Engineering-in-Medicine-and-Biology-Magazine 15: 100-104).

Weiterhin ist bekannt, daß Biomoleküle elektrostatisch, bspw. über Oberflächen mit polaren Gruppen, oder biochemisch, bspw. vermittels (Strept-)Avidin-beschichteter Träger, gebunden werden können.

So findet z.B. das (Strept-)Avidin aufgrund seiner Eigenschaft, Biotinmoleküle spezifisch zu binden, Verwendung, um die mit Biotin versehenen Moleküle mit dem beschichteten Träger zu koppeln. Dadurch ist es möglich, Oligonukleotide, Lipide, Oligo- und Polysacchsaride oder Peptide, die mit Biotin oder Biotinderivaten markiert sind, an die (Strept-)Avidin-Schicht des Trägers zu koppeln. (Liddell, E. und Weeks, I: 1996: Antikörpertechniken, Spektrum Akademischer Verlag Heidelberg, S.87-107).

Bekannte Testsysteme zur Überprüfung der inhibitorischen Wirkung von Substanzen gegenüber den Bewegungsvorgängen von Motorproteinen basieren auf dem Gesamtzell-Assay (Mayer, Th. U., Kapoor, T. M., Haggarty, S. J., King, R. W., Schreiber, S. L. and TT. J. Mitchison, 1999: Small molecule inhibitor of mitotic spindel bipolarity identified in a phenotype-based screen, Science, 286: 971-974), und dem Gleitassay (Sakowicz, R., Berdelis, M. S., Ray, K., Blackburn, Ch. L. Hopmann, C., Faulkner, D. J. and L. S. B Goldstein, 1998, A marin natural product inhibitor of kinesin motors, Science, 280: 292-295; Böhm, K. J., Steinmetzer, P., Daniel, A., Baum, M. and E. Unger, 1997: Kinesin-driven microtubule motitity in the presence of alkaline-earth metal ions. Indication for calcium ion-dependent motility. Cell Motility and Cytoskeleton 37: 226 - 231).

Der Nachteil dieser Testsysteme ist jedoch, daß sie sich manueller Verfahren bedienen, die äußerst arbeitsaufwendig, zeitintensiv, nicht parallelisierbar und somit kostenträchtig und für Screenings mit einem hohen Probendurchsatz anhand von Substanzbibliotheken nicht geeignet sind.

Ein automatisches Verfahren zur Auswertung des Gleitassays ist bspw. aus der Publikation von C. Götz und anderen (Götz, C., Uttenweiler, D., Streubing, R. und R. H. A. Fink, 1999, Molekulare Fluoreszenzmikroskopie zur Untersuchung von Motorproteinen, Bioforum 3: 98-100) bekannt, bei dem die Gleitgeschwindigkeit von Motorproteinen bestimmbar ist. Diese Verfahren senkt den Arbeitsaufwand und ist parallelisierbar, besitzt aber den Nachteil, daß der Zeitaufwand für die Gleitgeschwindigkeitsmessung nicht verringert werden kann und daß die Auswertung vermittels Fluoreszenzmikroskop und Bildverarbeitungstechnik erfolgt.

Die Schrift von Massow A. et al: "Interaction between Kinesin microtubules and microtubule-associated protein 2'cell motility and the cytoskeleton", Bd. 14, 1989, Seiten 562-571, offenbart ein Verfahren zum Nachweis der Wechselwirkungen zwischen Kinesinmolekülen und Mikrotubuli umfassend folgende Schritte:
a) eine Affinitätsschicht wird mit Kinesinmolekülen beschichtet;
b) eine Mikrotubuli enthaltende Lösung wird mit der Affinitätsschicht in Kontakt gebracht; und
c) die Bewegung der Mikrotubuli entlang der Kinesinmoleküle wird mit einem Mikroskop nachgewiesen.

Zusammenfassend kann festgestellt werden, daß eine Vielzahl von molekularbiologischen Daten zu den Kinesinen (Motorproteinen) bekannt ist, aber aufgrund der aufwendigen Form der Aktivitätsmessung über die Bewegungshemmung in Gleitassay eine breit angelegte Suche nach neuen Inhibitoren nicht aufwandgering ohne Mikroskop möglich ist.

Der Erfindung liegt die Aufgabe zugrunde, Kinesin-spezifische Wirkstoffe, insbesondere Inhibitoren der Kinesin-Last- und Kinesin-Mikrotubulus-Bindungen, zu identifizieren.

Die Aufgabe wird durch die kennzeichnenden Merkmale des ersten Patentanspruchs gelöst. Vorteilhafte Ausgestaltungen sind durch die nachgeordneten Ansprüche erfaßt.

Das Wesen der Erfindung besteht darin, daß bei dem Verfahren ein Träger mit einer Vielzahl adressierbar beschick-, wasch- und auslesbarer Probenaufnahmebereiche vorgesehen wird, wobei die Probenaufnahmebereiche zumindest in Teilbereichen mit einer Affinitätsschicht versehen werden, anschließend die Affinitätsschichten mit einer Lösung aus einem Wirkstoff und Kinesinmolekülen beschickt werden, wobei die Kinesinmolekülen mit einem physikalisch, chemisch oder biologisch nachweisbaren Marker versehen werden, ein Waschschritt durchgeführt und im Ergebnis dessen, der Verlust oder das Vorhandensein der mit dem Marker versehenen Kinesinmoleküle nachgewiesen wird.

Auf diese Art und Weise kann die inhibitorische Funktion von Wirkstoffen auf die Kinesin-Last-Bindung einfach detektiert werden, indem bspw. das Verfahren in einer Mikrotiterplatte durchgeführt wird und das Auslesen mit einem herkömmlichen Photometer erfolgt.

Alternativ dazu wird das Verfahren in der Art durchgeführt, daß im Anschluß an das Aufbringen der Affinitätsschichten diese mit Kinesinmolekülen beschichtet und nach einem Waschschritt mit einer Lösung aus einem Wirkstoff und Mikrotubuli beschickt werden, wobei die Mikrotubuli mit einem physikalisch, chemisch oder biologisch nachweisbaren Marker versehen werden, ein weiterer Waschschritt durchgeführt wird und im Ergebnis dessen der Verlust oder das Vorhandensein der mit dem Marker versehenen Mikrotubuli nachgewiesen wird.

Auf diese Art und Weise kann die inhibitorische Funktion von Wirkstoffen auf die Kinesin-Mikrotubuli-Bindung einfach detektiert werden, indem bspw. das Verfahren in einer Mikrotiterplatte durchgeführt wird und das Auslesen mit einem herkömmlichen Photometer erfolgt.

Die durch das Verfahren in den Probenaufnahmebereichen positionierten Affinitätsschichen werden als zu den Vesikelbindungsdomänen der Kinesinmoleküle affine Bindungssubstrate, bspw. in Form von Chromosomen oder Biomembranen bzw. deren molekularen Bestandteile oder in Form organischen Monoschichten, nach dem Stand der Technik auf den Träger aufgebracht.

Besonders vorteilhaft finden bei dem Verfahren negative Oberflächenladungen als Affinitätsschicht Verwendung, was die Durchführung des Verfahrens erleichtert, da dadurch das Aufbringen der Affinitätsschicht in Form von Chromosomen, Biomembranen oder organischen Monoschichten entfällt.

Neben den Kinesinmolekülen können auch spezifisch modifizierte Kinesinmoleküle für das Verfahren verwendet werden. Diese modifizierten Moleküle, die immer noch die Funktion der Lastbindungsdomänen aufweisen, werden nach dem Stand der Technik, bspw. als mikrobielle Fusionsproteine, gentechnisch hergestellt, indem die Motordomänen- und Mikrotubulusbindungsdomänen eliminiert werden.

Die Kinesinmoleküle (oder die modifizierten Kinesinmoleküle) werden direkt mit Markern gekoppelt, die besonders vorteilhaft fluoreszierend sind. Möglich ist aber auch, entsprechend dem Stand der Technik, andere Marker zu verwenden.

Alternativ zur direkten Markierung können die Kinesinmoleküle (oder die modifizierten Kinesinmoleküle) gemäß dem Stand der Technik auch indirekt mit den Markern in der Art versehen werden, daß die Marker über spezifische Antikörper an die Kinesinmoleküle (oder die modifizierten Kinesinmoleküle) gekoppelt werden, was vorteilhaft für die Untersuchnung der Kinesin-Last-Bindung durchgeführt wird.

Zur Untersuchnung der Kinesin-Mikrotubulus-Bindung werden bei dem erfindungsgemäßen Verfahren die Marker über die Mikrotubuli an die Kinesinmoleküle (oder die modifizierten Kinesinmoleküle) gekoppelt.

Die Markierung der Mikrotubuli erfolgt dabei nach dem Stand der Technik, wodurch die Marker direkt oder indirekt an die Mikrotubuli gebunden werden.

Besonders vorteilhaft ist dabei gemäß dem Stand der Technik, die indirekten Kopplung der Marker über mikrotubulusspezifische Antikörper.

Da bei dem Verfahren als Marker fluoreszierende chemische Verbindungen eingesetzt werden, erfolgt die Detektion der Fluoreszenz der Marker durch optisches Auslesen der Probenaufnahmebereiche des Trägers vermittels eines klassischen Photometers, wie es bspw. zum Auslesen von Mikrotiterplatten Verwendung findet.

Eine Miniaturisierung des Auslesens kann auch in der Art erfolgen, daß der Träger ein Biochip ist, der auf seinen Spots gebunden die Kinesinmoleküle (oder die modifizierten Kinesinmoleküle) trägt und vermittels eines Auslesemoduls in Form CCD-Technologie kombiniert wird.

Da die Marker nicht nur fluoreszierende chemische Verbindungen sein können, kann die Detektion auch nach dem Stand der Technik mit anderen Auswerteverfahren vermittels eines Auslesmoduls erfolgen.

Die Wirkungsweise des erfindungsgemäßen Verfahrens basiert auf der Blockade bzw. dem Lösen der Verbindung zwischen der Affinitätsschicht und den Kinesinmolekülen (oder den modifizierten Kinesinmolekülen) in der Art, daß der Wirkstoff mit Inhibitorwirkung die Verbindung der Last-Bindungsdomäne zur Affinitätsschicht stört, so daß aufgrund dieser Interaktion eine ja/nein-Entscheidung getroffen werden kann, die ein schnelles, kostengünstiges Auslesen, bspw. vermittels Markereinsatz und Photometrie, ermöglicht, was gegenüber den bisher bekannten Tests, bspw. dem Gleitassay, einen großen Vorteil darstellt

Alternativ dazu können die Wirkstoffe mit Inhibitorfunktion, entsprechend ihrer Wirkungsweise, auch die Verbindung zwischen den Kinesinmolekülen (oder den modifizierten Kinesinmolekülen) und den Mikrotubuli blockieren bzw. lösen, so daß aufgrund der vorstehend beschriebenen ja/nein-Entscheidung auch in diesem Fall ein schnelles, kostengünstiges Auslesen möglicht ist.

Die Erfindung soll nachstehend anhand von Ausführungsbeispielen und den schematischen Zeichnungen näher erläutert werden. Es zeigen:
- Fig. 1:: eine zum Verfahren verwendete Ausführungsform eines Trägers zur Testung des Kinesin-Last-Komplexes und
- Fig. 2:: eine zum Verfahren verwendete Ausführungsform eines Trägers zur Testung des Kinesin-Mikrotubulus-Komplexes.

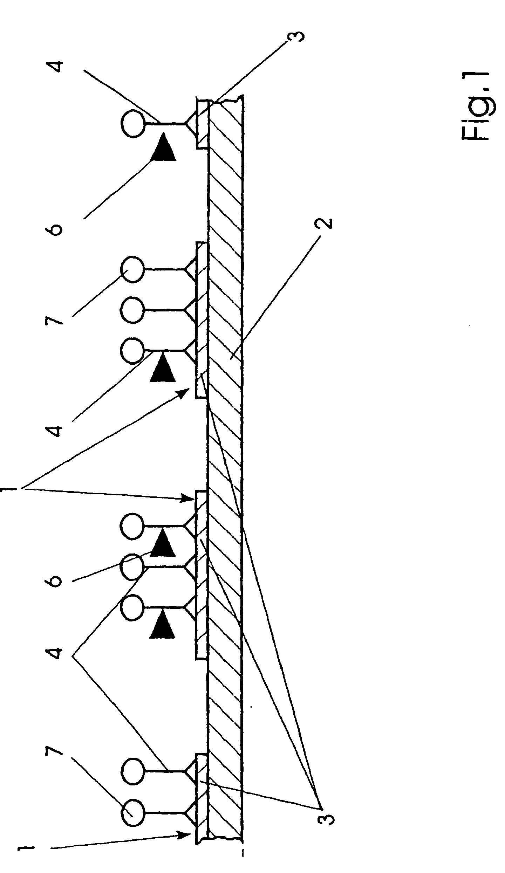

Das erfindungsgemäße Verfahren wird z.B. für die Testung des Kinesin-Last-Komplexes ausgeführt, indem ein in Fig. 1 dargestellter Träger 2 aus Glas oder in Mikrotiterplattenform verwendet wird, der negative Oberflächenladungen aufweist, durch die die Affinitätsschicht 3 ausgebildet wird. Diese Affinitätsschicht 3 wird in den Probenaufnahmebereichen 1 in einem definierten Raster vermittels eines Pipettierautomaten spotweise und Einzelproben zuordenbar mit einer Lösung beschickt. Diese Lösung wird aus einem Inkubationspuffer mit pH 6,8 bestehend aus 20 mM Pipes, 1 mM EGTA, 80 mM NaCl, 0,5 mM MgCl₂, 1 mM DTT sowie 100 bis 500 µg/ml Kinesin 4 und verschiedenen Einzelproben verschiedenster Wirkstoffe in einer Konzentration im nM- bis mM-Bereich zusammengesetzt.

Das Kinesin 4, im Beispiel das konventionelle neuronale Kinesin, ist dabei mit Oregon Green 514, einem Fluoreszenzfarbstoff als Marker 7, gekoppelt. Die Kopplung erfolgt in diesem Fall gemäß dem Stand der Technik vor dem Zusatz des Kinesins 4 zur Lösung.

Alternativ zu dieser Markierung wird ein modifiziertes Kinesin (Chromokinesin) hergestellt, indem gemäß dem Stand der Technik aus einem Klon (Accession N°AJ271784.1), der eine Deletion des Bereichs aufweist, der für den die Motordomäne und die Mikrotubulusdomäne tragenden N-Terminus des Kinesins 4 kodiert, die Plasmid-DNA isoliert und mit dem für das Grün-Fluoreszierende-Protein kodierende Plasmid (GFP-Plasmid) gekoppelt wird sowie dieses Kopplungsprodukt in *E.coli* gemäß dem Stand der Technik exprimiert wird. Das gebildete Fusionsprotein (modifiziertes Kinesin 4 mit direkt gekoppelten Marker 7) wird gemäß dem Stand der Technik isoliert. Alternativ zu dieser direkten Markierung kann das Kinesin 4, nach dem Stand der Technik, auch indirekte mit dem Marker 7 gekoppelt werden, bspw. über Antikörper.

Nach einer Inkubationszeit von 5 bis 15 min mit der zuvor beschriebenen Lösung werden die Probenaufnahmebereiche vermittels des Pipettierautomaten drei mal mit Inkubationspuffer zuzüglich 10 µM Taxol gespült.

Der Nachweis der vorhandenen markierten, bzw. der Nachweis des Verlustes des markierten Kinesins infolge der inhibitorischen Funktion einzelner zu testender Wirkstoffe wird mit einem üblichen Photometer bei einer Wellenlänge von 511 nm (Green Oregon-Markierung) bzw. 395 nm GFP-Markierung) durchgeführt, so daß vermittels des Verfahrens und der Detektion gewonnenen ja/nein Aussagen bzgl. der Markers 7 Rückschlüsse zur inhibitorischen Funktion der getesteten Wirkstoffe getroffen werden.

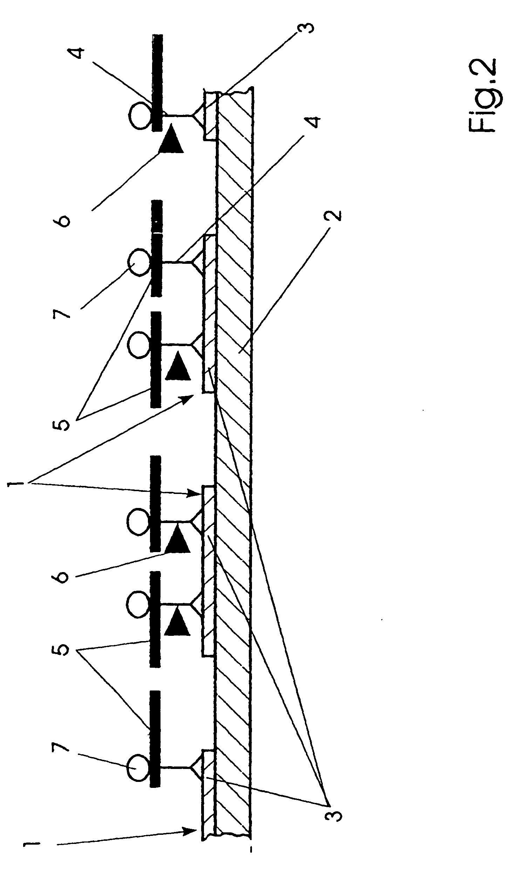

Für die Testung des Kinesin-Mikrotubus-Komplexes wird das Verfahren ausgeführt, indem ein in Fig. 2 dargestellter Träger 2 aus Glas oder in Mikrotiterplattenform verwendet wird, der negative Oberflächenladungen aufweist, durch die die Affinitätsschicht 3 ausgebildet wird und diese Affinitätsschicht 3 in den Probenaufnahmebereichen 1 vermittels eines Pipettierautomaten spotweise mit einer Lösung beschickt wird. Diese Lösung wird aus einem Inkubationspuffer mit pH 6,8 bestehend aus 20 mM Pipes, 1 mM EGTA, 80 mM NaCl, 0,5 mM MgCl₂, 1 mM DTT sowie 100 bis 500 µg/ml Kinesin zusammengesetzt. Das Kinesin 4 wird dabei im Beispiel durch das konventionelle neuronale Kinesin gebildet. Nach der Kinesinanbindung, die 5 bis 15 min lang durchgeführt wird, werden die Probenaufnahmebereiche 1 vermittels des Pipettierautomaten drei mal mit Inkubationspuffer gespült.

Nach diesem Spülen werden die Probenauthahmebereiche 1 in einem definierten Raster vermittels eines Pipettierautomaten spotweise und Einzelproben zuordenbar mit einer Lösung beschickt. Diese Lösung wird aus einem Inkubationspuffer mit pH 6,8 bestehend aus 20 mM Pipes, 1 mM EGTA, 80 mM NaCl, 0,5 mM MgCl₂, 1 mM DTT sowie 20 bis 50 µg/ml reassemblierter Mikrotubuli 5 und verschiedenen Einzelproben verschiedenster Wirkstoffe 7 in einer Konzentration im nM- bis mM-Bereich zusammengesetzt.

Die Mikrotubuli 5, im Beispiel in stabilisierenden Taxol aufgenommene neuronale Mikrotubuli, sind dabei mit Oregon Green 514, einem Fluoreszenzfarbstoff als Marker 7, gekoppelt (direkte Markierung). Die Kopplung erfolgt in diesem Fall gemäß dem Stand der Technik vor dem Zusatz des Mikrotubuli 5 zur Lösung. Alternativ dazu kann auch, gem. dem Stand der Technik, eine indirekte Kopplung des Markers 7, bspw. über Antikörper, erfolgen.

Nach einer Inkubationszeit von 5 bis 15 min mit der zuvor beschriebenen Lösung werden die Probenaufnahmebereiche 1 vermittels des Pipettierautomaten drei mal mit Inkubationspuffer zuzüglich 10 µM Taxol gespült.

Der Nachweis der vorhandenen markierten, bzw. der Nachweis des Verlustes der markierten Mikrotubuli 5 infolge der inhibitorischen Funktion einzelner zu testender Wirkstoffe wird mit einem üblichen Photometer bei einer Wellenlänge von 511 nm durchgeführt, so daß vermittels des Verfahrens und der Detektion gewonnenen ja/nein Aussagen bzgl. der Markers 7 Rückschlüsse zur inhibitorischen Funktion der getesteten Wirkstoffe getroffen werden.

### Bezugszeichenliste:

- 1: - Probenaufnahmebereich
- 2: - Träger
- 3: - Affinitätsschicht
- 4: - Kinesinmoleküle
- 5: - Mikrotubuli
- 6: - Wirkstoff
- 7: - Marker

## Patentansprüche

1. Verfahren zur Identifikation von Kinesin-spezifischen Wirkstoffen bei dem
a) eine Affinitätsschicht entweder mit zu den Vesikelbindungsdomänen der Kinesinmoleküle affinen Bindungsmolekülen oder mit Kinesinmolekülen beschichtet wird;
b) eine Lösung, die entweder einen zu testenden Wirkstoff und markierte Kinesinmoleküle oder einen zu testenden Wirkstoff und markierte Mikrotubuli enthält, mit der Affinitätsschicht in Kontakt gebracht wird; und
c) das von dem Marker erzeugte Signal nachgewiesen wird.

2. Verfahren zur Identifikation von Kinesin-spezifischen Wirkstoffen nach Anspruch 1, **dadurch gekennzeichnet, dass** als Bindungsmoleküle Chromosomen oder deren molekulare Bestandteile verwendet werden.

3. Verfahren zur Identifikation von Kinesin-spezifischen Wirkstoffen nach Anspruch 1, **dadurch gekennzeichnet, dass** als Bindungsmoleküle Biomembranen oder deren molekulare Bestandteile verwendet werden.

4. Verfahren zur Identifikation von Kinesin-spezifischen Wirkstoffen nach Anspruch 1, **dadurch gekennzeichnet, dass** als Bindungsmoleküle organische Monoschichten verwendet werden.

5. Verfahren zur Identifikation von Kinesin-spezifischen Wirkstoffen nach Anspruch 1, **dadurch gekennzeichnet, dass** als Affinitätsschicht negative Oberflächenladungen verwendet werden.

6. Verfahren zur Identifikation von Kinesin-spezifischen Wirkstoffen nach Anspruch 1, **dadurch gekennzeichnet, dass** als Kinesinmoleküle modifizierte Kinesinmoleküle eingesetzt werden, denen ihre Motordomänen und / oder Mikrotubulibindungsdomänen fehlen.

7. Verfahren zur Identifikation von Kinesin-spezifischen Wirkstoffen nach den Ansprüchen 1 und 7, **dadurch gekennzeichnet, dass** als modifizierte Kinesinmoleküle gentechnisch modifizierte, heterolog exprimierte Proteine eingesetzt werden.

8. Verfahren zur Identifikation von Kinesin-spezifischen Wirkstoffen nach Anspruch 1, **dadurch gekennzeichnet, dass** die Marker indirekt vermittels spezifischer Antikörper an die Kinesinmoleküle oder Mikrotubuli gebunden werden.

9. Verfahren zur Identifikation von Kinesin-spezifischen Wirkstoffen nach den Ansprüchen 1 oder 9, **dadurch gekennzeichnet, dass** als Marker fluoreszierende chemische Verbindungen eingesetzt werden.

## Claims

1. Method for detecting kinesin-specific active ingredients in which
a) an affinity layer is loaded either with bonding molecules that are affine to the vesicle-bonding domains of the kinesin molecules or with kinesin molecules,
b) a solution that either contains an active ingredient to be tested and marked kinesin molecules or an active ingredient to be tested and marked microtubules is brought into contact with the affinity layer, and
c) the signal produced by the marker is proven.

2. Method for detecting kinesin-specific active ingredients according to claim 1, wherein chromosomes or their molecular components are used as bonding molecules.

3. Method for detecting kinesin-specific active ingredients according to claim 1, wherein bio-membranes or their molecular components are used as bonding molecules.

4. Method for detecting kinesin-specific active ingredients according to claim 1, wherein organic monolayers are used as bonding molecules.

5. Method for detecting kinesin-specific active ingredients according to claim 1, wherein negative surface charges are used as the affinity layer.

6. Method for detecting kinesin-specific active ingredients according to claim 1, wherein modified kinesin molecules that are lacking their motor domains and / or their microtubule-bonding domains are used as kinesin molecules.

7. Method for detecting kinesin-specific active ingredients according to claims 1 and 7, wherein genetically modified, heterologously expressed proteins are used as bonding molecules.

8. Method for detecting kinesin-specific active ingredients according to claim 1, wherein the markers are indirectly bound to the kinesin molecules or microtubules by means of specific antibodies.

9. Method for detecting kinesin-specific active ingredients according to claims 1 and 9, wherein fluorescent chemical compounds are used as markers.

## Revendications

1. Procédé de détection de l'activité de substances actives spécifiques pour la kinésine qui prévoit
a) d'appliquer sur une couche assurant l'affinité une couche comprenant ou des molécules de liaison affines aux domaines de liaison pour vésicules ou des molécules de kinésine ;
b) de mettre en contact avec la couche d'affinité une solution comprenant soit une substance active à tester et des molécules de kinésine marqués soit une substance active à tester et des microtubules marquées ;
c) de fournir la preuve de l'existence du signal produit par le marqueur.

2. Le procédé de détection de l'activité de substances actives spécifiques pour la kinésine est **caractérisé en ce que** des chromosomes ou leurs composants moléculaires font office de molécules liants.

3. Le procédé de détection de l'activité de substances actives spécifiques pour la kinésine est **caractérisé en ce que** des membranes biologiques ou leurs composants moléculaires font office de molécules liants.

4. Le procédé de détection de l'activité de substances actives spécifiques pour la kinésine est **caractérisé en ce que** des monocouches organiques font office de molécules liantes.

5. Le procédé de détection de l'activité de substances actives spécifiques à la kinésine suivant la revendication 1 est **caractérisé en ce que** des charges superficielles négatives font office de couche d'affinité.

6. Le procédé de détection de l'activité de substances actives spécifiques pour la kinésine suivant la revendication 1 est **caractérisé en ce que** sont appliquées, en tant que molécules de kinésine, des molécules modifiées de kinésine dépourvues de leurs domaines moteurs et/ou de leurs domaines de liaison de microtubules.

7. Le procédé de détection de l'activité de substances actives spécifiques pour la kinésine suivant les revendications 1 et 7 est **caractérisé en ce que** des protéines d'expression hétérologue et génétiquement modifiées font office de molécules de kinésine.

8. Le procédé de détection de l'activité de substances actives spécifiques pour la kinésine suivant la revendication 1 est **caractérisé en ce que** les marqueurs sont liés au molécules de kinésine ou aux microtubules moyennant des anticorps spécifiques.

9. Le procédé de détection de l'activité de substances actives spécifiques pour la kinésine suivant les revendications 1 ou 9 est **caractérisé en ce que** des liaisons chimiques fluorescentes font office de marqueurs.
